# EUROPEAN PATENT APPLICATION

(11) **EP 3 748 009 A1**
(43) Date of publication of application: **09.12.2020**
(21) Application number: 19178973.4
(22) Date of filing: 07.06.2019
(51) Int. Cl.: C12P 17/04, C12P 41/00, C07D 307/33

(54) **SYNTHESIS OF (R)-PANTOLACTONE ACETATE**

(71) Applicant: DSM IP Assets B.V., 6411 TE Heerlen (NL)
(72) Inventor: Bekerle-Bogner, Myria, 8010 Graz (AT); Medlock, Jonathan Alan, 4303 Kaiseraugst (CH); Steinkellner, Georg, 8010 Graz (AT); Strohmeier, Gernot, 8010 Graz (AT); Steiner, Kerstin, 8010 Graz (AT)
(74) Representative: Seibel-Thomsen, Nadja

(57) **Abstract**

The present invention relates to a 2-step process comprising step (1) kinetic resolution of R/S-pantolactone using an enantioselective acetylating enzyme and step (2) racemization of (S)-pantolactone in the presence of at least one specific chemical catalyst leading to highly enriched (R)-pantolactone acetate. Both step (1) and (2) might be carried-out in a one-pot reaction/system.

## Description

The present invention relates to a 2-step process comprising step (1) kinetic resolution of R/S-pantolactone using an enantioselective acetylating enzyme and step (2) racemization of (S)-pantolactone in the presence of at least one specific chemical catalyst leading to highly enriched (R)-pantolactone acetate. Both step (1) and (2) might be carried-out in a one-pot reaction/system.

Pantothenate or vitamin B5 is a member of the B complex of vitamins which is essential for mammals including humans. It has to be supplied as food or feed additive. In cells, pantothenate is used primarily for the biosynthesis of coenzyme A and acyl carrier protein. These essential coenzymes function in the metabolism of acyl moieties, which form thioesters with the sulfhydryl group of the 4'-phosphopantethein portion of these molecules. Importantly, only (R)-pantothenate is biologically active as vitamin.

Pantothenate has been synthesized conventionally via chemical synthesis from bulk chemicals. However, the substrates required for chemical synthesis are expensive and the racemic intermediates have to be optically resolved. Bacterial or microbial systems, if available, that produce enzymes useful in pantothenate biosynthesis processes, are not yet feasible for industrial production because of insufficient yield and/or selectivity of the used enzymes.

Thus, there is a need for more effective and simpler methods towards enantioselective production of (R)-pantolactone to be used in an industrial scale, with no need for further resolution and/or purification steps.

Surprisingly, we now identified a process wherein racemic pantolactone together with an acetate ester can be converted into pantolactone acetate, with a percentage of up to 100% in the (R)-conformation, said process comprising the steps of (1) enantioselective alcoholysis of (R/S)-pantolactone and an acetate ester using (R)-selective enzymes and (2) racemization of (S)-pantolactone using a specific transition metal complex as (chemical) catalyst. The (R)-pantolactone acetate can be further converted into (R)-pantolactone by methods known in the art.

Particularly, the present invention is directed to (1) a process for conversion of racemic (R/S)-pantolactone via catalytic action of enzymes having carboxylic ester hydrolase [EC 3.1.1] activity, including triacylglycerol lipase enzymes [EC 3.1.1.3] and cutinase enzymes [EC 3.1.1.74], wherein (R/S)-pantolactone and an acetate ester are selectively converted into (R)-pantolactone-acetate via enantioselective acetylation under non-aqueous conditions.

For the purpose of the present invention, i.e. enantioselective acetylation of racemic pantolactone and an acetate ester into (R)-pantolactone acetate as of step (1) and as described herein, any enzyme with activity of α/β-hydrolases, particularly enzymes of class [EC 3.1.1], isolated from various sources can be used, particularly such enzymes isolated from fungi including yeast, bacteria or higher eukaryotes. Preferably, the enzyme is selected from the class [EC 3.1.1.3] or [EC 3.1.1.74], including but not limited to Candida antarctica lipase A (CalA) including CalA-like enzymes isolated from Kurtzmanomyces sp. (NCBI accession code 20429169), Malassezia furfur (NCBI accession code 73765555) or Ustilago maydis (NCBI accession code 71018653), cutinase 1 (Cut1) or other lipases, such as e.g. isolated from Thermomyces lanuginosus (Humicola lanuginosa), Fusarium solani, Humicola insolens, Candida antarctica including C. antarctica lipase B (CalB), or Rhizomucor miehei.

In one embodiment, the enantioselective enzyme to be used in step (1) and as defined herein is a lipase, such as e.g. selected from Fusarium solani, preferably F. solani pisi cutinase 1 (Cut1), more preferably a polypeptide with at least about 80%, such as e.g. 85, 90, 95, 98 or 100% identity to SEQ ID NO:1, including polynucleotides encoding said polypeptide such as e.g. the polynucleotide of SEQ ID NO:2. Cut1 is commercially available from various suppliers, e.g. such as ASA Spezialenzyme GmbH (Germany).

In one embodiment, the enantioselective enzyme to be used in step (1) and as defined herein is selected from Candida, preferably Candida antarctica lipase B (CalB), more preferably a polypeptide with at least about 80%, such as e.g. 85, 90, 95, 98 or 100% identity to SEQ ID NO:3, including polynucleotides encoding said polypeptide such as e.g. the polynucleotide of SEQ ID NO:4. CalB is commercially available from various suppliers, e.g. such as Novozymes or Merck.

In one embodiment, the enantioselective enzyme to be used in step (1) and as defined herein is a lipase, such as e.g. selected from Thermomyces lanuginosus (Humicola lanuginosa), preferably Thermomyces lanuginosus (Humicola lanuginosa) lipase (TLL), more preferably a polypeptide with at least about 80%, such as e.g. 85, 90, 95, 98 or 100% identity to SEQ ID NO:5, including polynucleotides encoding said polypeptide such as e.g. the polynucleotide of SEQ ID NO:6. TLL is commercially available from various suppliers, e.g. such as Novozymes or Merck.

The term "α/β-hydrolase", "lipase", or "enzyme having lipase activity", "pantolactone hydrolyzing enzyme", "acetylating enzyme", "alcoholysis enzyme", "hydrolysis enzyme", "enantioselective enzyme", "(R)-selective enzyme" or "(R/S)-pantolactone transesterase" are used interchangeably herein. It refers to an enzyme having lipase or α/β-hydrolase activity which is involved in conversion, i.e. enantioselective hydrolysis (alcoholysis/acetylation), of (R/S)-pantolactone and an acetate ester into (R)-pantolactone acetate as defined herein. The terms "CalB" and "Candida antarctica lipase B", "TLL" and "Thermomyces lanuginosus lipase", or "Cut1" and "Fusarium solani pisi cutinase 1" are used interchangeably herein.

Suitable acetate esters to be used for acetylation of (R/S)-pantolactone are selected from either unsaturated, saturated or aromatic acetates including but not limited to vinyl acetate, phenylvinyl acetate, ethoxyvinyl acetate, isoprenyl acetate, isopropenyl acetate, ethyl acetate or p-chlorophenyl acetate, preferably vinyl acetate.

The enzymatic conversion of (R/S)-pantolactone and an acetate ester as defined herein into (R)-pantolactone acetate, i.e. enantioselective hydrolysis/acetylation, as described herein can be performed with isolated enzymes, such as e.g. the acetylating enzymes as defined herein, particularly lipases or cutinases, such as e.g. CalB, Cut1, TLL, that might be expressed in a suitable host cell, such as e.g. yeast or bacteria, preferably Pichia or Escherichia, such as e.g. as heterologous enzymes. Particularly, the enantioselective enzymes as described herein are heterologous expressed in Pichia pastoris. Isolation of suitable enzymes can be done by known methods, the respective enzymes might be further used in isolated form or in the form of a cell extract, powder or liquid formulations, i.e. in immobilized or non-immobilized form. Preferably, the enzymes - either modified or non-modified - as defined herein, such as e.g. CalB, Cut1, TLL, that are used in a process for enantioselective acetylation of (R/S)-pantolactone and an acetate ester as defined herein, are used in isolated form, such as e.g. in liquid or immobilized form, such as either covalently bound or adsorbed, e.g. on epoxy carrier, depending on the supplier. When using immobilized forms, the enzymes might be used several times with more or less the same performance (so-called recycling). The described enzyme forms apply to both the modified and non-modified enzymes.

The terms "conversion", "hydrolysis", "alcoholysis", "acetylation", "enantioselective acetylation", "enantioselective hydrolysis" or "enantioselective alcoholysis" in connection with step (1), i.e. enzymatic catalysis of the enzymes [EC 3.1.1] as described herein, preferably enzymes selected from class [EC 3.1.1.3] or [EC 3.1.1.74], leading to enantioselective production of (R)-pantolactone acetate from racemic pantolactone and an acetate ester as defined herein, are used interchangeably herein. As used herein, the term "conversion rate" refers to the activity of the α/β-hydrolases, particularly CalB, Cut1, TLL, which can be measured as conversion of (R/S)-pantolactone into pantolactone acetate. The term "E value" (E) as used herein refers to the general enantioselectivity of the enzyme independent of the conversion rate. The "enantiomeric excess" (ee) as used herein refers to the degree to which a sample contains one enantiomer (such as e.g. (R)-pantolactone) in higher amount than the other enantiomer (such as e.g. (S)-pantolactone), see Helpfile on selectivity, Uni Graz, 2012: http://biocatalysis.uni-graz.at/enantio/DataFiles/Selectivity-Help.pdf.

The term "racemic (R/S)-pantolactone" refers to a mixture comprising (R) and (S)-pantolactone in any ratio, including a mixture wherein the (R) and (S)-enantiomers are present in equal quantities, i.e. a so-called racemate.

As used herein, the term "specific activity" or "activity" with regards to enzymes means its catalytic activity, i.e. its ability to catalyze formation of a product from a given substrate. The specific activity defines the amount of substrate consumed and/or product produced in a given time period and per defined amount of protein at a defined temperature. Typically, specific activity is expressed in µmol substrate consumed or product formed per min per mg of protein. Typically, µmol/min is abbreviated by U (= unit). Therefore, the unit definitions for specific activity of µmol/min/(mg of protein) or U/(mg of protein) are used interchangeably throughout this document. An enzyme is active, if it performs its catalytic activity in vivo, i.e. within the host cell as defined herein or within an in vitro system in the presence of a suitable substrate. The skilled person knows how to measure enzyme activity, in particular activity of hydrolysis enzymes as defined herein, such as in particular CalB, Cut1, TLL activity. Analytical methods to evaluate the capability of a suitable enzyme as defined herein for formation of (R)-pantolactone acetate from hydrolysis of (R/S)-pantolactone and an acetate ester as defined herein are known in the art.

The present invention is directed to a process according to step (1) for enantioselective hydrolysis of (R/S)-pantolactone, wherein the (R/S)-pantolactone and an acetate ester as defined herein is enzymatically converted into pantolactone acetate and (S)-pantolactone with a conversion rate in the range of at least about 20, such as e.g. at least about 25, 30, 35, 40, 45, 50, 55, or up to 60 % conversion of racemic pantolactone into pantolactone acetate. Preferably, the pantolactone acetate is present as (R)-enantiomer, with preferred E values of at least about 10, such as e.g. 15, 20, 25, 30, 40, 50, 60 or even 100 and more.

In a particular embodiment, the process according to the present invention comprising step (1) and step (2) leads to a conversion of (R/S)-pantolactone and an acetate ester as defined herein into (R)-pantolactone acetate with a conversion rate of at least about 20, such as e.g. at least about 25, 30, 35, 40, 45, 50, 55, 60, 65, 70, 75, 80, 85, 90, 95 or even up to 100% and E values of at least about 10, such as e.g. 15, 20, 25, 30, 40, 50, 60 or even 100 and more.

In a way of further improving the enantioselectivity towards (R)-pantolactone acetate according to step (1) and as defined herein, particularly for improving the conversion rate and/or E values of said lipases and cutinases as defined herein, one or more amino acid modification(s) might be introduced, preferably into the substrate binding region of the enzymes. Particularly and exemplified herein are modified cutinase enzymes including but not limited to Cut1, preferably Cut1 from Fusarium solani pisi. These results might be applicable to other Cut1 enzymes from other organisms or respective lipases as defined herein.

Thus, the present invention is directed to a hydrolase as defined herein that has been modified via the introduction of one or more amino acid substitution(s) in a sequence corresponding to amino acid residues 28 to 169 in the polypeptide with at least about 80% identity to SEQ ID NO:1, such as e.g. substitution(s) of one or more amino acid(s) in a sequence corresponding to residues T28 to L169 in the polypeptide according SEQ ID NO:1, more particularly via introduction of one or more amino acid substitution(s) at position(s) corresponding to residue(s) selected from the group consisting of position 28, 66, 169, and combinations thereof in the polypeptide with at least about 80%, such as e.g. 85, 90, 95, 98 or 100% identity to SEQ ID NO:1, preferably leading to leucine or alanine on position 28, and/or alanine on position 66 and/or 169. Using such modified enzymes for enantioselective hydrolysis of (R/S)-pantolactone with an acetate ester according to step (1) as defined herein, conversion rates in the range of up to about 50% and more with E values in the range of up to about 42 and more can be achieved. These values might vary depending on both the solvent and the incubation time.

As used herein, a "modified" enzyme, e.g. modified lipase or cutinase, has a preferred activity and/or specificity towards enantioselective formation of (R)-pantolactone acetate compared to the corresponding reaction catalyzed by a non-modified enzyme. Preferred enzyme is Cut1, such as Cut1 from Fusarium solani pisi. A "non-modified" enzyme as defined herein, particularly Cut1, as used herein refers to the respective endogenous or commercially available enzymes not carrying one or more amino acid substitution(s) as defined herein.

As used herein, a host cell carrying/expressing a modified catalytic activity as defined herein, particularly Cut1, comprising one or more amino acid substitution(s) as defined herein, is referred to as "modified" host cell. The respective host cell carrying a non-modified catalytic activity, such as encoding the wild-type Cut1 gene, is referred to as "non-modified" host cell.

In one embodiment, the modified enzyme as defined herein, in particular enzyme with modified Cut1 activity, i.e. improved enantioselective activity towards (R)-pantolactone acetate, comprises an amino acid substitution at a position corresponding to residue 28 in the polypeptide according to SEQ ID NO:1, preferably substitution of threonine by lysine or alanine (T28L or T28A). Using said modified Cut1 enzyme in step (1) as defined herein, E values in the range of about 27 (with toluene as co-solvent) to about 42 (with EtOAc as co-solvent) after 18h at 40°C of reaction could be achieved, compared to step (1) using the respective non-modified enzyme, including but not limited to Cut1 according to e.g. SEQ ID NO:1, with E values of about 23 (with toluene as co-solvent) to about 33 (with methyl tert-butylether as co-solvent) after 18h at 40°C.

In one embodiment, the modified enzyme as defined herein, in particular enzyme with modified Cut1 activity, i.e. improved enantioselective activity towards (R)-pantolactone acetate, comprises an amino acid substitution at a position corresponding to residue 66 in the polypeptide according to SEQ ID NO:1, preferably substitution of lysine by alanine (L66A). Using said modified Cut1 enzyme in step (1) as defined herein, E values in the range of at least about 25 (with toluene as co-solvent) to about 38 (with EtOAc as co-solvent) after 18h at 40°C of reaction could be achieved, compared to step (1) using the respective non-modified enzyme, including but not limited to Cut1 according to e.g. SEQ ID NO:1, with E values of at least about 23 (with toluene as co-solvent) to about 33 (with MTBE as co-solvent) after 18h at 40°C.

In a further embodiment, the modified enzyme as defined herein, in particular enzyme with modified Cut1 activity, i.e. improved enantioselective activity towards (R)-pantolactone acetate, comprises an amino acid substitution at a position corresponding to residue 169 in the polypeptide according to SEQ ID NO:1, preferably substitution of valine by alanine (V169A). Using said modified Cut1 enzyme in step (1) as defined herein, E values in the range of at least about 27 (with toluene as co-solvent) to about 38 (with methyl tert-butylether as co-solvent) after 18h at 40°C of reaction could be achieved, compared to step (1) using the respective non-modified enzyme, including but not limited to Cut1 according to e.g. SEQ ID NO:1, with E values of at least about 23 (with toluene as co-solvent) to about 33 (with methyl-tert-butylether as co-solvent) after 18h at 40°C.

The described amino acid substitution(s) at a position corresponding to residues as disclosed herein might be combined with each other, leading to conversion rates and/or an E value which are increased as compared to single mutations, such as e.g. an increase in the range of at least about 2, 5, 10, 20, 50% compared to single mutations. Selectivity mutations in enzyme active sites often have an additive or preferably synergistic effect when they are combined, thus increasing the overall selectivity of the enzyme. Thus, combination of the selectivity mutations described herein might improve the E value of the enzymes even more, such as e.g. leading to E values of about 10 or more and/or conversion rates in the range of about 50% or more.

The generation of a mutation into nucleic acids or amino acids, i.e. mutagenesis, may be performed in different ways, such as for instance by random or site-directed mutagenesis, physical damage caused by agents such as for instance radiation, chemical treatment, or insertion of a genetic element. The skilled person knows how to introduce mutations.

In one aspect of the present invention, a host cell comprising and expressing a modified or non-modified enantioselective enzyme as defined herein, such as e.g. modified or non-modified Cut1 activity, i.e. improved enantioselective activity towards (R)-pantolactone acetate, is used in a process for production of (R)-pantolactone acetate from conversion of racemic (R/S)-pantolactone and an acetate ester as defined herein. The host cell comprising the modified/non-modified enzyme may be cultured in an aqueous medium supplemented with appropriate nutrients under aerobic or anaerobic conditions and as known by the skilled person for the respective host cells. Optionally, such cultivation is in the presence of proteins and/or co-factors involved in transfer of electrons, as known in the art. The cultivation/growth of the host cell may be conducted in batch, fed-batch, semi-continuous or continuous mode. (R)-pantolactone acetate might be isolated and/or optionally further purified from the mix of (R)-pantolactone acetate and (S)-pantolactone.

In one embodiment of the invention, step (1) as described herein is carried out using an immobilized modified or non-modified enzyme for enantioselective acetylation of (R/S)-pantolactone with an acetate ester to mainly form (R)-pantolactone acetate. Such process might be performed under conditions known in the art, e.g. such as in an aqueous medium. Particularly, said process according to step (1) is performed under conditions which are not interfering with the racemization of (S)-pantolactone of step (2). Examples of suitable solvents which could be used in step (1) include but are not limited to aliphatic hydrocarbons, aromatic hydrocarbons, chlorinated hydrocarbons, esters, ethers, and/or alcohols, particularly under conditions comprising at least about 1% of water. Immobilization might be performed with Lifetech ECR8285 (Purolite® Life Sciences) or other methods know to the skilled person. The amount of enzyme used according to step (1) might vary, such as in the range of at least about 2, 5, 6, 8, 10, 12, 15, 18, 20 mg enzyme per mL solvent (dry immobilized enzyme in mass%), such as e.g. 10 to 17 mg/mmol pantolactone, with reactions carried out at about 35-45°C, such as e.g. about 40°C.

With regards to the present invention, it is understood that organisms, such as e.g. microorganisms, fungi, algae or plants also include synonyms or basonyms of such species having the same physiological properties, as defined by the International Code of Nomenclature of Prokaryotes or the International Code of Nomenclature for algae, fungi, and plants (Melbourne Code).

The present invention is directed to a 2-step process, wherein (R/S)-pantolactone and an acetate ester are selectively converted into (R)-pantolactone-acetate via enantioselective acetylation and as described above as step (1) and wherein the by-product (S)-pantolactone is converted back into (R/S)-pantolactone in the presence of one or more specific transition metal complex(es) as (chemical) catalyst(s) and as described herein as step (2).

As used herein, the term "conversion" in connection with step (2) refers to the activity of the transition metal complex used for selective racemization of (S)-pantolactone into (R/S)-pantolactone. The term "racemization" is art-recognized and refers to conversion of either (R)- or (S)-enantiomers in a racemic form of (R/S)-isomers. As used herein, it refers to the selective conversion of (S)-pantolactone into (R/S)-pantolactone.

In one embodiment, the present invention is directed to a process comprising step (1) and step (2), wherein both steps are performed in one reaction, i.e. in one-pot. They might be alternatively performed in a loop or cascade reactor. In case step (1) and (2) are performed in a loop or cascade reactor, the temperature in step (1) and (2) might vary, e.g. with step (1) being carried out at about 40°C and step (2) being carried out at higher temperature, e.g. at least about 50°C. With regards to step (1), the reaction time might vary, with durations of about 18 to 72h or up to 258h are possible.

Particularly, the racemization of (S)-pantolactone according to step (2) is performed under conditions which are not interfering with the enzymatic acetylation of step (1) and vice-versa.

In a particular embodiment, the racemization of step (2) and as described herein is carried out in the presence of at least one specific transition metal complex as (chemical) catalyst, wherein the transition metal(s) is/are selected from the group consisting of Ru, Rh, Ir, Fe, Mn and Co, preferably selected from Ru or Ir. The at least one transition metal complex might comprise one or more ligands. In a preferred embodiment, a catalyst to be used in step (2) and as defined herein is the "Shvo catalyst", which is 1-hydroxytetraphenyl-cyclopentadienyl(tetraphenyl-2,4-cyclopentadien-1-one)-µ-hydrotetracarbonyldiruthenium(II). This catalyst was first described by Shvo et al. (J.Am.Chem.Soc., 1986, 108, pp. 7400-7402).

A suitable solvent for the racemization as of step (2) and as described herein might be selected from aliphatic hydrocarbons, aromatic hydrocarbons, chlorinated hydrocarbons, esters, ethers, and alcohols, preferably selected from THF, 2-methyl-THF, toluene, xylene, cyclohexane, or ethyl acetate. Solvents to be used in a one-pot reaction as described herein wherein step (1) is performed in the presence of immobilized enzymes might be selected from organic solvents as described above, such as e.g. toluene, dichloromethane, acetone, methyl tert-butylether (MTBE), ethyl acetate and the like.

The process according to step (2) and as described herein might be carried out at elevated temperatures, such as e.g. temperatures up to 100°C, such as e.g. between about 25 to 100°C, preferably between about 40 to 90°C. With regards to step (1) and (2) carried out in a one-pot reaction, the temperature might be selected from about 25 to 80°C, preferably from about 30 to 70°C, such as 40°C, but particularly preferred at least about 50°C.

The process comprising step (1) and (2) as described herein is used for production of (R)-pantolactone acetate. The (R)-pantolactone acetate is furthermore hydrolyzed to (R)-pantolactone and converted to pantothenic acid and its derivatives by well-known transformations.

The following examples are illustrative only and are not intended to limit the scope of the invention in any way.

### Examples

### Example 1: General methods, media, strains and plasmids

All basic molecular biology and DNA manipulation procedures described herein were generally performed according to Sambrook et al. (1989. Molecular Cloning: A Laboratory Manual. Cold Spring Harbor Laboratory Press: New York) or Ausubel et al. (1998. Current Protocols in Molecular Biology. Wiley: New York).

### Example 2: Expression of mutant enzymes in Pichia pastoris

Genes encoding Cut1, CalB or TLL variants, i.e. modified Cut1, CalB or TLL were codon-harmonized and cloned into the methanol inducible Pichia pastoris expression vector pAaHswa (Ahmad et al., Appl Microbiol Biotechnol (2014) 98:5301-5317).

Single colonies from a selection plate were inoculated into 2.5 ml BMGY (200 rpm, 25°C). On the 5^{th} day, 2.083 ml BMMY was added to each well, with further shaking at 200 rpm (30°C). After 6 h, 21 µl MeOH was added to each well. This addition was repeated again after further 16, 24, 40, and 48 h. After 8 days in total, the cells were harvested (10 min, 1000xg, 4°C), the supernatant transferred to an Eppendorf tube and stored at 4°C.

For detection of active protein in the culture supernatant, activity of the variants on the model substrate para-nitrophenyl butyrate (p-NPB) was measured. The assay monitors the product formation of para-nitrophenol (p-NP) over time using a UV/VIS spectrophotometer. All variants showed activity towards conversion into (R)-pantolactone acetate.

### Example 3: Enantioselective acetylation of (R/S)-pantolactone

Formation of (R)-pantolactone acetate was tested with the best Cut1-variants (see Table 2), all used as immobilized enzymes (Purolite® Lifetech™ ECR8285), wherein proteins were covalently bound on a carrier. Reactions were performed in closed glass-vials in the presence of vinyl acetate and various organic solvents in excess together with the tested enzyme-preparations at 40°C. At specific time points, a sample was taken to analyse the probes via chiral gas chromatography. The conversion rates as well as E values were defined.

Chiral GC-FID-measurements were done on an Agilent HP 6890 gas chromatograph equipped with hot-split injector, autosampler and FID detector. Column used was Chiralsil-Dex 25 m x 0.32 mm, 0.25 µm film (9082223, #CP7503, Varian). The inlet temperature was 200°C, detector temperature was 220°C, with 1.0 ml/min constant N2 flow, split ratio 10:1, 110°C hold 10 min, 10°C/min to 160°C hold 2 min. Retention times are listed below:

**Table 1. Retention times (tᵣₑₜ) as measured according to the method described in the text.**

| tᵣₑₜ [min] | | | |
|---|---|---|---|
| (*R*)-pantolactone acetate | (*S*)-pantolactone acetate | (*S*)-pantolactone | (*R*)-pantolactone |
| 11.16 | 11.51 | 12.65 | 13.17 |

**Table 2: Improved (R)-selectivity of Cut1 variants obtained from Cut1 from Fusarium solani pisi. (1) 500 mM DL-1, 2.0 M vinyl acetate, 10 mg/mL (20 mg/mmol 1) Fusarium solani pisi cutinase (wt or variants) on Purolite® LifetechTM ECR8285, 40°C; (2) determined by chiral GC analysis on a Chiralsil-Dex column; excess enantiomer mentioned in parentheses; (3) calculated according to the equations shown above; E value: excess (R)-enantiomer of (2; (4) Conversions calculated based on peak areas.**

| Enzyme variant | Co-solvent | Reaction time [h] | ee (S) of (1) [%] | ee (R) of 2a | E value | Conversion [%] |
|---|---|---|---|---|---|---|
| T28A | MTBE | 18 | 13 | 94 | 40 | 13 |
| | | 72 | 29 | 93 | 36 | 24 |
| | | 258 | 54 | 90 | 31 | 39 |
| | EtOAc | 18 | 11 | 95 | 42 | 10 |
| | | 72 | 20 | 93 | 34 | 18 |
| | | 258 | 34 | 91 | 29 | 28 |
| | acetone | 18 | 6 | 94 | 38 | 6 |
| | | 72 | 4 | 93 | 27 | 4 |
| | | 258 | 7 | 88 | 17 | 8 |
| | toluene | 18 | 6 | 92 | 27 | 6 |
| | | 72 | 17 | 91 | 24 | 16 |
| | | 258 | 37 | 87 | 21 | 30 |
| T28L | MTBE | 18 | 17 | 94 | 40 | 16 |
| | | 72 | 41 | 93 | 40 | 31 |
| | | 258 | 79 | 88 | 38 | 50 |
| | EtOAc | 18 | 14 | 94 | 39 | 13 |
| | | 72 | 28 | 93 | 35 | 23 |
| | | 258 | 52 | 89 | 50 | 38 |
| | acetone | 18 | 8 | 94 | 37 | 8 |
| | | 72 | 17 | 93 | 31 | 16 |
| | | 258 | 30 | 91 | 27 | 25 |
| | toluene | 18 | 8 | 93 | 28 | 8 |
| | | 72 | 21 | 91 | 26 | 19 |
| | | 258 | 38 | 88 | 23 | 31 |
| L66A | MTBE | 18 | 6 | 94 | 33 | 6 |
| | | 72 | 26 | 92 | 29 | 15 |
| | | 258 | 34 | 90 | 27 | 27 |
| | EtOAc | 18 | 5 | 95 | 38 | 4 |
| | | 72 | 9 | 93 | 30 | 9 |
| | | 258 | 17 | 90 | 23 | 16 |
| | acetone | 18 | 2 | 95 | 38 | 2 |
| | | 72 | 8 | 93 | 31 | 8 |
| | | 258 | 13 | 91 | 24 | 12 |
| | toluene | 18 | 3 | 92 | 25 | 4 |
| | | 72 | 7 | 89 | 18 | 8 |
| | | 258 | 15 | 84 | 14 | 15 |
| V169A | MTBE | 18 | 14 | 94 | 38 | 13 |
| | | 72 | 32 | 93 | 36 | 26 |
| | | 258 | 64 | 89 | 35 | 43 |
| | EtOAc | 18 | 8 | 93 | 32 | 8 |
| | | 72 | 12 | 93 | 33 | 11 |
| | | 258 | 18 | 91 | 26 | 17 |
| | acetone | 18 | 4 | 94 | 33 | 4 |
| | | 72 | 10 | 92 | 26 | 9 |
| | | 258 | 15 | 90 | 21 | 15 |
| | toluene | 18 | 9 | 92 | 27 | 9 |
| | | 72 | 19 | 91 | 25 | 17 |
| | | 258 | 39 | 87 | 21 | 31 |
| wt | MTBE | 18 | 16 | 93 | 33 | 15 |
| | | 72 | 39 | 91 | 32 | 30 |
| | | 258 | 75 | 87 | 29 | 49 |
| | EtOAc | 18 | 11 | 93 | 30 | 11 |
| | | 72 | 15 | 93 | 34 | 14 |
| | | 258 | 26 | 92 | 30 | 22 |
| | acetone | 18 | 5 | 93 | 30 | 5 |
| | | 72 | 12 | 90 | 21 | 12 |
| | | 258 | 24 | 86 | 17 | 22 |
| | toluene | 18 | 10 | 91 | 23 | 10 |
| | | 72 | 23 | 89 | 21 | 21 |
| | | 258 | 48 | 84 | 18 | 37 |

**Table 3: Sequence of Cut1-wt, CalB-wt and TLL-wt. The mutated amino acid residues in Cut1 are indicated in bold. SEQ ID NO:1 and 3 show the mature polypeptide, whereas SEQ ID NO:5 still includes the signal sequence (underlined).**

| SEQ ID NO: | Name of oligo or enzyme | sequence 5' to 3' |
|---|---|---|
| 1 | Cut1 | GRTTRDDLINGNSASCRDVIFIYARGSTETGNLGTLGPSIASNLESAFGKDGV WIQGVGGAYRATLGDNALPRGTSSAAIREMLGLFQQANTKCPDATLIAGGYSQ GAALAAASIEDLDSAIRDKIAGTVLFGYTKNLQNRGRIPNYPADRTKVFCNTG DLVCTGSLIVAAPHLAYGPDARGPAPEFLIEKVRAVRGSA |
| 2 | cut1 | GCCAGCATTGCTGCTAAAGAAGAAGGGGTATCTCTCGAGAAAAGAGAGGCTGA AGCTGGTAGAACTACCAGGGACGATTTGATCAACGGTAACTCCGCTTCCTGCA GGGACGTTATTTTCATCTACGCTAGAGGTTCCACCGAGACTGGTAACTTGGGT ACTTTGGGTCCATCCATTGCTTCCAACTTGGAATCCGCTTTCGGTAAGGACGG TGTCTGGATTCAAGGTGTTGGTGGTGCTTACAGAGCTACCTTGGGTGATAACG CTTTGCCAAGAGGTACTTCCTCCGCTGCTATTAGAGAGATGTTGGGTTTGTTC CAGCAGGCCAACACTAAGTGTCCAGACGCTACTTTGATTGCCGGTGGTTACTC TCAAGGTGCTGCTTTGGCTGCTGCTTCTATTGAAGATTTGGACTCCGCCATCA |
| | | GAGACAAGATCGCTGGTACTGTTTTGTTCGGTTACACCAAGAACCTGCAGAAC AGAGGTAGAATCCCAAACTACCCAGCCGACAGAACTAAGGTTTTCTGTAACAC TGGTGACCTGGTCTGTACCGGTTCTTTGATAGTTGCTGCTCCACACTTGGCTT ACGGTCCAGATGCTAGAGGTCCAGCTCCAGAATTCTTGATCGAAAAGGTTAGA GCCGTCAGAGGTTCTGCTTAAGCGGCCGCAAACGGTTTTAGCCTTAGACATGA CTGTTC |
| 3 | CalB | |
| 4 | calB | GGTATCTCTCGAGAAAAGAGAGGCTGAAGCTTTGCCATCTGGTTCTGATCCAG CTTTCTCACAGCCAAAGTCTGTTTTGGACGCTGGTTTGACTTGTCAAGGTGCT TCTCCATCTTCCGTGTCCAAGCCAATTTTGTTGGTTCCAGGTACTGGTACTAC TGGTCCACAATCTTTCGACTCCAACTGGATCCCATTGTCCACTCAGTTGGGTT ACACTCCATGTTGGATTTCCCCACCACCATTCATGTTGAACGACACCCAAGTT AACACCGAGTACATGGTTAACGCTATCACTGCCTTGTACGCTGGTTCCGGTAA CAACAAGTTGCCAGTTTTGACTTGGTCCCAAGGTGGTTTGGTTGCTCAATGGG GTTTGACATTCTTCCCATCCATCAGATCCAAGGTCGACAGATTGATGGCTTTC GCTCCAGACTACAAGGGTACTGTTTTGGCTGGTCCATTGGATGCTTTGGCAGT TTCTGCTCCATCCGTTTGGCAACAAACTACTGGTTCCGCTTTGACTACCGCTT TGAGAAACGCTGGTGGTTTGACCCAAATCGTTCCAACCACTAACTTGTACTCC GCTACCGACGAAATCGTTCAGCCACAAGTTTCTAACTCCCCACTGGACTCCTC TTACCTGTTCAACGGAAAGAACGTTCAGGCTCAGGCTGTTTGTGGTCCACTGT TCGTTATTGACCATGCTGGTTCCTTGACTTCCCAGTTCTCTTACGTTGTTGGT AGATCCGCCTTGAGATCCACTACTGGACAAGCTAGATCTGCCGACTACGGTAT CACTGACTGTAACCCATTGCCAGCTAACGACTTGACTCCAGAGCAAAAGGTTG CTGCTGCAGCTTTATTGGCTCCAGCTGCTGCTGCTATAGTTGCTGGTCCAAAG CAAAACTGTGAGCCAGACTTGATGCCATACGCTAGACCATTTGCCGTTGGAAA GAGAACCTGTTCCGGTATCGTTACTCCATAAGCGGCCGCAAACGGT |
| 5 | TLL | MRSSLVLFFVSAWTALASPIRREVSQDLFNQFNLFAQYSAAAYCGKNNDAPAG TNITCTGNACPEVEKADATFLYSFEDSGVGDVTGFLALDNTNKLIVLSFRGSR SIENWIGNLNFDLKEINDICSGCRGHDGFTSSWRSVADTLRQKVEDAVREHPD YRVVFTGHSLGGALATVAGADLRGNGYDIDVFSYGAPRVGNRAFAEFLTVQTG GTLYRITHTNDIVPRLPPREFGYSHSSPEYWIKSGTLVPVTRNDIVKIEGIDA TGGNNQPNIPDIPAHLWYFGLIGTCL |
| 6 | tLL | GCCAGCATTGCTGCTAAAGAAGAAGGGGTATCTCTCGAGAAAAGAGAGGCTGA AGCTGAGGTTTCCCAGGACTTGTTTAACCAGTTCAACCTGTTCGCTCAGTACT CCGCTGCTGCTTACTGTGGTAAGAACAACGATGCTCCTGCCGGAACTAACATC ACTTGTACTGGTAACGCTTGTCCAGAGGTTGAAAAGGCTGACGCTACTTTCTT GTACTCCTTCGAGGATTCTGGTGTCGGTGACGTTACTGGTTTCTTGGCTTTGG ACAACACCAACAAGTTGATCGTCCTGTCCTTCAGAGGTTCCAGATCCATTGAG AACTGGATCGGTAACCTGAACTTCGACCTGAAAGAGATCAACGACATCTGCTC CGGTTGTAGAGGTCACGATGGTTTCACTTCATCTTGGAGATCCGTTGCCGACA CCTTGAGACAAAAGGTTGAGGACGCTGTTAGAGAGCACCCAGACTACAGAGTT GTTTTTACCGGTCACTCTCTTGGTGGTGCTTTGGCTACTGTTGCTGGTGCTGA TTTGAGAGGTAACGGTTACGACATCGACGTGTTCTCTTACGGTGCTCCAAGAG TTGGTAACAGAGCTTTCGCTGAGTTCTTGACTGTTCAGACTGGTGGAACCCTG TACAGAATCACTCACACTAACGACATCGTCCCAAGATTGCCACCTAGAGAATT |
| | | CGGTTACTCTCACTCTTCCCCAGAATACTGGATCAAGTCCGGTACTTTGGTTC CAGTCACCAGAAACGACATTGTCAAGATCGAGGGTATCGACGCTACCGGTGGT AACAACCAGCCAAACATTCCAGATATCCCAGCTCACTTGTGGTACTTCGGTTT GATCGGTACTTGCTTGTAAGCGGCCGCAAACGGTTTTAGCCTTAGACATGACT GTTC |

### Example 4: Racemization of (S)-pantolactone according to step (2)

(S)-Pantolactone can be obtained from the mixture obtained in step (1) (see Ex. 3) by separation from (R)-pantolactone acetate. Alternatively, the mixture of (S)-pantolactone and (R)-pantolactone acetate from step (1) (see Ex. 3) can be used directly.

(S)-Pantolactone (310-345 mg, 2.4-2.6 mmol) was placed in a 10 ml round bottomed flask with the "Shvo catalyst" (5-10 mol%). The solvent (3 ml) was added and the mixture was inertised. The reaction mixture was heated at the described temperature for 16-20h, cooled and analysed by GC (column: Chirasil-DEX CB (25 m x 0.25 mm; Film 0.25 µm), temperature program: 130°C (constant)). The result is shown in Table 4.

**Table 4: Reactions starting from (S)-pantolactone (i.e. a mixture comprising a ratio of (R) : (S) = 2 : 98). "Exp." means "experiment", with Exp.1a given as comparative example.**

| Exp. # | Catalyst [mol%] | Temp [°C] | Solvent | Ratio (R):(S) pantolactone |
|---|---|---|---|---|
| 1a | none | 65 | THF | 2 : 98 |
| 1b | 5 | 75 | toluene | 47 : 53 |
| 1c | 5 | 75 | cyclohexane | 49 : 59 |
| 1d | 5 | 80 | toluene | 49 : 51 |
| 1e | 5 | 55 | EtOAc | 37 : 68 |
| 1f | 5 | 65 | THF | 25 : 75 |

Examples 3 and 4 can be combined together and carried out in a one-pot reaction, in a loop or cascade reactor with step (1) and (2) taking place at different sections (areas) of the loop.

## Claims

**1.** A process for production of (R)-pantolactone acetate comprising the steps of (1) enantioselective alcoholysis of (R/S)-pantolactone and an acetate ester using (R)-selective enzymes having carboxylic ester hydrolase [EC 3.1.1] activity, preferably including triacylglycerol lipase enzymes [EC 3.1.1.3] and cutinase enzymes [EC 3.1.1.74], preferably with a conversion rate of at least about 20%, and (2) racemization of (S)-pantolactone in the presence of at least one specific transition metal complex as (chemical) catalyst.

**2.** The process according to claim 1, wherein the enzyme of step (1) is selected from the group consisting of Kurtzmanomyces sp., Malassezia furfur, Ustilago maydis, Thermomyces lanuginosus (Humicola lanuginosa), Fusarium solani, Humicola insolens, Candida antarctica, and Rhizomucor miehei, preferably an enzyme with at least about 80%, such as e.g. 85, 90, 95, 98 or 100% identity to SEQ ID NOs:1, 3, 5.

**3.** An enantioselective alcoholysis enzyme catalyzing the conversion of racemic pantolactone and an acetate ester into (R)-pantolactone acetate according to step (1) in claim 1 or 2, said enzyme having cutinase enzyme [EC 3.1.1.74] activity and an E value of at least about 10.

**4.** The enzyme according to claim 3, said enzyme comprising one or more amino acid substitution(s) in the substrate binding region.

**5.** The enzyme according to claim 4, said one or more amino acid substitution(s) are located at position(s) corresponding to residue(s) selected from the group consisting of position 28, 66, 169, and combinations thereof in the polypeptide according to SEQ ID NO:1.

**6.** The enzyme according to claim 5, wherein the one or more amino acid substitution(s) are selected from T28L, T28A, L66A, V169A, and combinations thereof, corresponding to residue(s) selected from the group consisting of position 28, 66, 169, and combinations thereof in the polypeptide according to SEQ ID NO:1.

**7.** A host cell, preferably a yeast, more preferably Pichia, comprising a heterologous enzyme according to any one of claims 3 to 6.

**9.** Use of an enzyme according to any one of claims 3 to 6 or a host cell according to claim 7 in a process for production of (R)-pantolactone acetate from racemic pantolactone and an acetate ester according to step (1) in claim 1 or 2, with an E value of at least about 10.

**10.** A process according to any one of claims 1, 2 or 8, comprising the step of racemization of (S)-pantolactone in the presence of at least one specific transition metal complex as catalyst, preferably wherein the at least one transition metal is selected from the group consisting of Ru, Rh, Ir, Fe, Mn and Co, more preferably selected from Ru or Ir.

**11.** The process according to claim 10, wherein the process is carried out in a solvent selected from the group consisting of aliphatic hydrocarbons, aromatic hydrocarbons, chlorinated hydrocarbons, esters, ethers, and alcohols, preferably selected from the group consisting of methyl tert-butylether, THF, 2-methyl-THF, toluene, xylene, cyclohexane, and ethyl acetate.

**12.** The process according to claim 10 or 11 wherein the process is carried out at temperatures between about 25 to 100°C, preferably between about 40 to 90°C.

**13.** The process according to any one of claims 11 to 13, comprising racemization of (S)-pantolactone in the presence of the Shvo catalyst, which is 1-hydroxytetraphenyl-cyclopentadienyl(tetraphenyl-2,4-cyclopentadien-1-one)-µ-hydrotetracarbonyldiruthenium(II) .

**14.** The process according to any one of claims 1, 2 or 9 to 14, wherein both step (1) and step (2) are performed in a one-pot reactor or in a loop or cascade reactor.


SEQUENCE LISTING

<110>  DSM IP Assets B.V.

<120>  Synthesis of (R)-pantolactone acetate

<130>  33350-EP-EPA

<160>  6

<170>  PatentIn version 3.5

<210>  1
<211>  199
<212>  PRT
<213>  Fusarium solani

<400>  1

Gly Arg Thr Thr Arg Asp Asp Leu Ile Asn Gly Asn Ser Ala Ser Cys
1               5                   10                  15

Arg Asp Val Ile Phe Ile Tyr Ala Arg Gly Ser Thr Glu Thr Gly Asn
            20                  25                  30

Leu Gly Thr Leu Gly Pro Ser Ile Ala Ser Asn Leu Glu Ser Ala Phe
        35                  40                  45

Gly Lys Asp Gly Val Trp Ile Gln Gly Val Gly Gly Ala Tyr Arg Ala
    50                  55                  60

Thr Leu Gly Asp Asn Ala Leu Pro Arg Gly Thr Ser Ser Ala Ala Ile
65                  70                  75                  80

Arg Glu Met Leu Gly Leu Phe Gln Gln Ala Asn Thr Lys Cys Pro Asp
                85                  90                  95

Ala Thr Leu Ile Ala Gly Gly Tyr Ser Gln Gly Ala Ala Leu Ala Ala
            100                 105                 110

Ala Ser Ile Glu Asp Leu Asp Ser Ala Ile Arg Asp Lys Ile Ala Gly
        115                 120                 125

Thr Val Leu Phe Gly Tyr Thr Lys Asn Leu Gln Asn Arg Gly Arg Ile
    130                 135                 140

Pro Asn Tyr Pro Ala Asp Arg Thr Lys Val Phe Cys Asn Thr Gly Asp
145                 150                 155                 160

Leu Val Cys Thr Gly Ser Leu Ile Val Ala Ala Pro His Leu Ala Tyr
                165                 170                 175

Gly Pro Asp Ala Arg Gly Pro Ala Pro Glu Phe Leu Ile Glu Lys Val
            180                 185                 190

Arg Ala Val Arg Gly Ser Ala
        195

<210>  2
<211>  695
<212>  DNA
<213>  Fusarium solani

<400>  2
gccagcattg ctgctaaaga agaaggggta tctctcgaga aaagagaggc tgaagctggt      60

agaactacca gggacgattt gatcaacggt aactccgctt cctgcaggga cgttattttc     120

atctacgcta gaggttccac cgagactggt aacttgggta ctttgggtcc atccattgct     180

tccaacttgg aatccgcttt cggtaaggac ggtgtctgga ttcaaggtgt tggtggtgct     240

tacagagcta ccttgggtga taacgctttg ccaagaggta cttcctccgc tgctattaga     300

gagatgttgg gtttgttcca gcaggccaac actaagtgtc cagacgctac tttgattgcc     360

ggtggttact ctcaaggtgc tgctttggct gctgcttcta ttgaagattt ggactccgcc     420

atcagagaca agatcgctgg tactgttttg ttcggttaca ccaagaacct gcagaacaga     480

ggtagaatcc caaactaccc agccgacaga actaaggttt tctgtaacac tggtgacctg     540

gtctgtaccg gttctttgat agttgctgct ccacacttgg cttacggtcc agatgctaga     600

ggtccagctc cagaattctt gatcgaaaag gttagagccg tcagaggttc tgcttaagcg     660

gccgcaaacg gttttagcct tagacatgac tgttc                                695

<210>  3
<211>  317
<212>  PRT
<213>  Candida antarctica

<400>  3

<210>  4
<211>  1000
<212>  DNA
<213>  Candida antarctica

<400>  4
ggtatctctc gagaaaagag aggctgaagc tttgccatct ggttctgatc cagctttctc      60

acagccaaag tctgttttgg acgctggttt gacttgtcaa ggtgcttctc catcttccgt     120

gtccaagcca attttgttgg ttccaggtac tggtactact ggtccacaat ctttcgactc     180

caactggatc ccattgtcca ctcagttggg ttacactcca tgttggattt ccccaccacc     240

attcatgttg aacgacaccc aagttaacac cgagtacatg gttaacgcta tcactgcctt     300

gtacgctggt tccggtaaca acaagttgcc agttttgact tggtcccaag gtggtttggt     360

tgctcaatgg ggtttgacat tcttcccatc catcagatcc aaggtcgaca gattgatggc     420

tttcgctcca gactacaagg gtactgtttt ggctggtcca ttggatgctt tggcagtttc     480

tgctccatcc gtttggcaac aaactactgg ttccgctttg actaccgctt tgagaaacgc     540

tggtggtttg acccaaatcg ttccaaccac taacttgtac tccgctaccg acgaaatcgt     600

tcagccacaa gtttctaact ccccactgga ctcctcttac ctgttcaacg gaaagaacgt     660

tcaggctcag gctgtttgtg gtccactgtt cgttattgac catgctggtt ccttgacttc     720

ccagttctct tacgttgttg gtagatccgc cttgagatcc actactggac aagctagatc     780

tgccgactac ggtatcactg actgtaaccc attgccagct aacgacttga ctccagagca     840

aaaggttgct gctgcagctt tattggctcc agctgctgct gctatagttg ctggtccaaa     900

gcaaaactgt gagccagact tgatgccata cgctagacca tttgccgttg gaaagagaac     960

ctgttccggt atcgttactc cataagcggc cgcaaacggt                          1000

<210>  5
<211>  291
<212>  PRT
<213>  Thermomyces lanuginosus

<400>  5

Met Arg Ser Ser Leu Val Leu Phe Phe Val Ser Ala Trp Thr Ala Leu
1               5                   10                  15

Ala Ser Pro Ile Arg Arg Glu Val Ser Gln Asp Leu Phe Asn Gln Phe
            20                  25                  30

Asn Leu Phe Ala Gln Tyr Ser Ala Ala Ala Tyr Cys Gly Lys Asn Asn
        35                  40                  45

Asp Ala Pro Ala Gly Thr Asn Ile Thr Cys Thr Gly Asn Ala Cys Pro
    50                  55                  60

Glu Val Glu Lys Ala Asp Ala Thr Phe Leu Tyr Ser Phe Glu Asp Ser
65                  70                  75                  80

Gly Val Gly Asp Val Thr Gly Phe Leu Ala Leu Asp Asn Thr Asn Lys
                85                  90                  95

Leu Ile Val Leu Ser Phe Arg Gly Ser Arg Ser Ile Glu Asn Trp Ile
            100                 105                 110

Gly Asn Leu Asn Phe Asp Leu Lys Glu Ile Asn Asp Ile Cys Ser Gly
        115                 120                 125

Cys Arg Gly His Asp Gly Phe Thr Ser Ser Trp Arg Ser Val Ala Asp
    130                 135                 140

Thr Leu Arg Gln Lys Val Glu Asp Ala Val Arg Glu His Pro Asp Tyr
145                 150                 155                 160

Arg Val Val Phe Thr Gly His Ser Leu Gly Gly Ala Leu Ala Thr Val
                165                 170                 175

Ala Gly Ala Asp Leu Arg Gly Asn Gly Tyr Asp Ile Asp Val Phe Ser
            180                 185                 190

Tyr Gly Ala Pro Arg Val Gly Asn Arg Ala Phe Ala Glu Phe Leu Thr
        195                 200                 205

Val Gln Thr Gly Gly Thr Leu Tyr Arg Ile Thr His Thr Asn Asp Ile
    210                 215                 220

Val Pro Arg Leu Pro Pro Arg Glu Phe Gly Tyr Ser His Ser Ser Pro
225                 230                 235                 240

Glu Tyr Trp Ile Lys Ser Gly Thr Leu Val Pro Val Thr Arg Asn Asp
                245                 250                 255

Ile Val Lys Ile Glu Gly Ile Asp Ala Thr Gly Gly Asn Asn Gln Pro
            260                 265                 270

Asn Ile Pro Asp Ile Pro Ala His Leu Trp Tyr Phe Gly Leu Ile Gly
        275                 280                 285

Thr Cys Leu
    290

<210>  6
<211>  905

<212>  DNA
<213>  Thermomyces lanuginosus

<400>  6
gccagcattg ctgctaaaga agaaggggta tctctcgaga aaagagaggc tgaagctgag      60

gtttcccagg acttgtttaa ccagttcaac ctgttcgctc agtactccgc tgctgcttac     120

tgtggtaaga acaacgatgc tcctgccgga actaacatca cttgtactgg taacgcttgt     180

ccagaggttg aaaaggctga cgctactttc ttgtactcct tcgaggattc tggtgtcggt     240

gacgttactg gtttcttggc tttggacaac accaacaagt tgatcgtcct gtccttcaga     300

ggttccagat ccattgagaa ctggatcggt aacctgaact tcgacctgaa agagatcaac     360

gacatctgct ccggttgtag aggtcacgat ggtttcactt catcttggag atccgttgcc     420

gacaccttga gacaaaaggt tgaggacgct gttagagagc acccagacta cagagttgtt     480

tttaccggtc actctcttgg tggtgctttg gctactgttg ctggtgctga tttgagaggt     540

aacggttacg acatcgacgt gttctcttac ggtgctccaa gagttggtaa cagagctttc     600

gctgagttct tgactgttca gactggtgga accctgtaca gaatcactca cactaacgac     660

atcgtcccaa gattgccacc tagagaattc ggttactctc actcttcccc agaatactgg     720

atcaagtccg gtactttggt tccagtcacc agaaacgaca ttgtcaagat cgagggtatc     780

gacgctaccg gtggtaacaa ccagccaaac attccagata tcccagctca cttgtggtac     840

ttcggtttga tcggtacttg cttgtaagcg gccgcaaacg gttttagcct tagacatgac     900

tgttc                                                                 905